# EUROPEAN PATENT APPLICATION

(11) **EP 4 011 212 A1**
(43) Date of publication of application: **15.06.2022**
(21) Application number: 21209787.7
(22) Date of filing: 15.07.2011
(51) Int. Cl.: A23C 9/15, A23C 9/146, A23L 2/66, A23C 9/142, A23J 3/08, B01D 15/08, A23L 33/19

(54) **DAIRY PRODUCT AND PROCESS**

(30) Priority: 16.07.2010 NZ 10586848
(62) Divisional of application: 11807119.0
(71) Applicant: Fonterra Co-Operative Group Limited, Auckland 1010 (NZ)
(72) Inventor: BHASKAR, Ganugapati Vijaya, Palmserton North (NZ); GAO, Hongping, Palmserton North (NZ); DONK, Rochelle Kathleen, Palmerston North (NZ)
(74) Representative: Forresters IP LLP

(57) **Abstract**

The invention relates to a liquid nutritional composition comprising (a) 2-25% by weight of a calcium-depleted milk protein concentrate (MPC) that has undergone a heat treatment to at least 80°C and has between 15-45% by weight of the calcium removed; (b) 0-30% by weight fat; (c) 5-45% by weight carbohydrate; wherein the nutritional composition has a viscosity of less than 200cP at a temperature of 20°C and shear rate of 100s⁻¹, and has an energy density of at least 0.5 kcal/ml, and wherein protein provides 10-40% of the total energy content of the composition. Also provided is a powdered composition dispersible in water to form the liquid composition.

## Description

### Field of the Invention

The invention relates to the application of a milk protein concentrate ingredient into high protein nutritional liquid foods and reconstitutable powders and their preparation.

### Background of the Invention

PCT published application WO2008/026940 discloses the use of a calcium-depleted milk protein ingredient that can be applied advantageously to the stabilisation of a variety of food products, especially fat containing foods. The dried calcium-depleted milk protein concentrate (MPC) ingredient may be applied in formulations at levels of 0.01% to 10% of the final food. Such foods are disclosed that contain a variety of fat, carbohydrate levels and varying amounts of minerals, vitamins, flavourants, etc. which are selected from the group, whole milk, buttermilk, filled and imitation milks, milk powders and filled milk powders, fat containing retentate powders, reconstituted milks, retentates and creams, coffee creamer and coffee whitener, ice-cream, infant formula, yoghurt (including set, stirred and drinking), mousse, soups, sauces, liqueurs, meat products, pet foods, mayonnaise, snack products, chocolate, confectionary, fat containing gels and the like.

The disclosed prior art calcium depleted MPC may be heat treated and may have at least 30% of the divalent cations replaced with mono-valent cations, e.g., Na or K ions and the ratio of casein to whey protein may be adjusted between 95:5 to 50:50 by weight.

The disclosed prior art calcium depleted MPC is described as being useful in the preparation of a "stabilised food or drink". The prior disclosure teaches that a "stabilised food or drink" is a food or drink that either or both has more texture or is more stable to separation into different phases than the corresponding food or drink without the calcium-depleted MPC.

A range of specialized foods (meal replacers and/or meal supplements) exist for elderly or convalescents or other patients that cannot get the nutrition required by eating normal foods or are unable to feed themselves or require assistance during feeding. Generic terms used to categorise these foods are "medical foods, enteral foods or enteral nutrition", i.e., foods that are taken under the supervision of a medical professional. In some jurisdictions medical foods / enteral nutrition has a legal definition. In the USA, the term medical food, as defined in section 5(b) of the Orphan Drug Act (21 U.S.C. 360ee (b) (3)) is "a food which is formulated to be consumed or administered enterally under the supervision of a physician and which is intended for the specific dietary management of a disease or condition for which distinctive nutritional requirements, based on recognized scientific principles, are established by medical evaluation". In some jurisdictions, such foods are available to the public only by prescription, in others they can be procured directly over the counter (OTC).

Enteral formulas are ingested both orally and through tubes. Oral ingestion is useful when nutrient supplements are necessary and both the digestive tract and the patient are capable of taking them. Tube feeding is necessary for patients who need supplements but cannot take nutrition orally.

All these foods have very exacting requirements. They require a high degree of heat treatment to provide sterility and long shelf life stability, high calorific density, i.e. highly concentrated doses of nutrients but at the same time low viscosity so that they can be readily administered to the patient and consumed easily.

Liquid nutritional foods are also used by healthy subjects as meal replacers or when a rapidly consumable feed is required. Liquid nutritional foods are generally suitable for use by the aged or by athletes.

It is an object of the invention to provide a low viscosity, high-energy density, high protein nutritional liquid food and/or a reconstitutable powder and/or to provide the public with a useful choice.

### Disclosure of Invention

In one aspect the invention provides a liquid nutritional composition comprising (a) 2-25% by weight of a calcium-depleted milk protein concentrate (MPC) that has undergone a heat treatment to at least 80°C and has 15-45% by weight of the calcium removed; (b) 0-30% by weight fat; (c) 5-45% by weight carbohydrate; wherein the nutritional composition has a viscosity of less than 200 cP at a temperature of 20°C and a shear rate of 100 s⁻¹, and has an energy density of at least 0.5 kcal/ml, and wherein protein provides 10-40% of the total energy content of the composition. The final composition is preferably heated to a temperature greater than 100°C.

In a second aspect the invention provides a powdered nutritional composition dispersible in water to form a liquid nutritional composition comprising (a) 2-25% by weight of a calcium-depleted milk protein concentrate (MPC) that has undergone a heat treatment to at least 80°C and has 15-45% by weight of the calcium removed; (b) 0-30% by weight fat; (c) 5-45% by weight carbohydrate; wherein the nutritional composition has a viscosity of less than 200 cP at 20°C and a shear rate of 100 s⁻¹, and has an energy density of at least 0.5 kcal/ml, and wherein protein provides 10-40% of the total energy content of the composition.

In a preferred embodiment the of the liquid composition, the calcium removed from milk protein concentrate (MPC) has been replaced by sodium or potassium; and the energy density is at least 1.5 kcal/ml, and protein provides 10-30% of the total energy content of the composition.

In another preferred embodiment the powdered composition is dispersible in water to form a liquid nutritional composition comprising (a) 2-25% by weight of a calcium-depleted milk protein concentrate (MPC) that has undergone a heat treatment to at least 80°C and has between 15-45% by weight of the calcium replaced by sodium or potassium; (b) 0-25% by weight fat; (c) 5-45% by weight carbohydrate; wherein the nutritional composition has a viscosity of less than 200cP, and has an energy density of at least 1.5 kcal/ml, and protein provides 10-30% of the total energy content of the composition.

The term "liquid nutritional composition" refers to an aqueous composition to be administered by mouth or by other means, generally by tube feeding, to the stomach or intestines of a patient. Such other means include naso-gastric feeding, gastronomy feeding, jejunostomy feeding, naso-duodenal and naso-jejunal feeding, and duodenostomy feeding. Liquid nutritional compositions include "medical foods", "enteral nutrition", "food for special medical purposes", liquid meal replacers and supplements. The liquid nutritional compositions of the present invention provide significant amounts of protein and carbohydrate and usually also fat. They may also include vitamins and minerals. In preferred embodiments they provide balanced meals.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

The term "milk protein concentrate" (MPC) refers to a milk protein product in which greater than 40%, preferably greater than 50%, more preferably greater than 55%, most preferably greater than 70% of the solids-not-fat (SNF) is milk protein (by weight) and the weight ratio of casein to whey proteins is between about 95:5 and about 50:50, preferably between 90:10 and 70:30, most preferably between 90:10 and 80:20. Such concentrates are known in the art. MPCs are frequently described with the % dry matter as milk protein being appended to "MPC". For example MPC70 is an MPC with 70% of the dry matter as milk protein. Generally MPCs are prepared by processes invoking ultrafiltration either to prepare a stream enriched in casein or a stream enriched in whey protein. The streams may be blended to attain desired ratios of casein to whey protein. In another embodiment, the milk protein concentrate may be prepared by blending a stream of skim milk with a stream of whey protein concentrate prepared by ultrafiltration, treating either the skim milk stream or the combined stream by cation exchange and optionally concentrating or drying.

Calcium depleted MPCs are MPCs in which the calcium content is lower than the corresponding non-depleted MPC. These products generally also have a lower content of other divalent cations, for example, magnesium, than corresponding non-depleted products. Generally for the purposes of this invention, references to calcium removal imply removal of other divalent cations including magnesium present in the milk, MPC, or nutritional composition unless indicated otherwise.

For the purpose of the present specification, the viscosity is measured at 20°C using a rheometer such as an Anton Paar instrument using a cup and bob assembly at a shear rate of 100s⁻¹, unless otherwise indicated.

For the purpose of the present specification, energy densities are as measured by calculation using standard calorific values of food constituents.

For the purposes of the present specification, protein concentrations are as measured by Kjeldahl analysis of total nitrogen.

The term "calcium ions" is used broadly and includes ionic calcium and colloidal calcium unless the context requires otherwise.

The term "magnesium ions" is used broadly and includes ionic magnesium and colloidal magnesium unless the context requires otherwise.

The term "charged substantially with a single species" indicates that a resin has at least 90% of the exchangeable ions as a single species, preferably at least 95%. In particular, the term indicates that resin is not prepared by mixing of resins bearing different species or that the resin has undergone a treatment calculated to provide charging with more than one type of ion. In this aspect of the invention it is contemplated, for example, that a small proportion of the cations bound to a cation exchange resin may be resistant to exchange with the desired cation.

The term "caseinate" refers to a chemical compound of casein and a metal ion produced by acid precipitation of casein followed by resolublisation with alkali comprising the metal ion. Standard MPC contains concentrated casein micelles. As a consequence of the calcium depletion treatment of this invention, the resulting calcium-depleted MPC ingredient does not contain original (native) casein micelles, but contains casein-calcium-sodium/potassium-phosphate complexes. These complexes are different from the original casein micelles, entirely due to the replacement of calcium by sodium/potassium. They are also different from calcium caseinate aggregates and sodium caseinate solutions in that the inventive complexes contain phosphate whereas caseinates contain substantially reduced phosphate levels arising from the acidic precipitation of the casein, its washing and subsequent resolublisation..

The applicant has found that the calcium depleted MPC ingredient can be applied advantageously in liquid nutritional compositions wherein the calcium depleted MPC confers the surprising benefit of low viscosity. The heat-treated calcium-depleted MPC ingredient is especially useful in that it provides low viscosity to medical, orally or enterally administered foods because it can be delivered readily by flow through a tube or by mouth.

Liquid nutritional foods are often calorifically dense in that they contain nutrients such as fat, protein and carbohydrates in levels and combinations to attain calorific values of at least 0.5 kcal/g or kcal/ml. In the group of medical or enteric foods calorific densities up to 3 kcal/g or even above are known. Such high calorific densities are difficult to achieve with low viscosity and sufficient protein.

Preferably the liquid nutritional composition comprises 5-20% protein, more preferably 5-15%. Preferably liquid nutritional composition comprises 4-25%, more preferably 4-20% of the heat-treated calcium-depleted MPC.

Preferably the fat content is 1-30% by weight, more preferably 5-20% most preferably between 5% and 15%.

Preferably the carbohydrate content is 5-40%, more preferably 10-35%, most preferably 20-30%.

The formulation of the liquid nutritional food may also contain a wide variety of vitamins and minerals required to sustain patients nutritionally for long periods of time, and minor components such as antioxidants, flavouring and colouring. The amounts of vitamins and minerals to be used are preferably those typical of meal replacement products known to those skilled in the art. The micro-nutritional requirements of various sub-groups of the population are also known.

Typically the dried non-fat ingredients are dispersed in water, allowed to hydrate, mixed and then mixed vigorously with fat In one embodiment the sugar (carbohydrate) and protein are mixed to assist in protein dispersion and solubilisation. Whilst protein and sugar (carbohydrate) mixes are the preferred method of dispersion and solubilisation, protein and fat mixes can also be used for improved dispersion and solubilisation.

The components of the composition of the invention are typically homogenised to reduce the fat/oil droplet size and form an oil-in-water emulsion and then heat treated to achieve sterility.

The mixing to form the stabilised food composition involves application of shear forces to reduce droplet size preferably to an average of less than 50 microns as categorised by the volume weighted average particle size parameter [D4,3], more preferably less than 20 microns, even more preferably less than 2 microns, most preferably less than 1 micron. For some embodiments high shear stirring, for example, in a blade mixer (for example an Ultra Turrax or Waring blender) may be used.

Two heat treatments may be used in the preparation of the liquid nutritional composition. The first treatment may be applied in the preparation of the calcium-depleted MPC. The second treatment is an optional heat treatment of the liquid nutritional composition after it has been prepared. The first treatment is necessary for giving the satisfactory solubility and heat stability in order to impart the required viscosity properties to the calcium-depleted MPC. The second is to increase storage time of the product and minimise the potential for growth of undesired microorganisms. Other known art non-thermal processes can be used to inhibit microbiological activity in the liquid nutritional composition.

The calcium-depleted MPC is preferably heated in the temperature range 80-140°C preferably 80-120°C, preferably held at a determined temperature for about for 1 second to 20 or more minutes. Shorter treating times may be used at higher temperatures.

Various heat treatments of the liquid nutritional composition may be used. Ultra-high heat treatment (UHT) is preferred. Typical UHT conditions are 140 to 150°C for 2 to 5 seconds. Another process used to ensure sterility is retort heat treatment - often 120-130°C for 10 to 20 minutes. Other combinations of equivalent heat treatment are known. To attain the required sterility, the proteins must be stable to the heat treatment conditions. The calcium depleted MPC ingredient has been found to be surprisingly stable to the required heat treatments. The heated liquid nutritional composition may be usefully homogenised (or re-homogenised) to stabilise the product. In an alternative embodiment, the homogenisation of the nutritional composition may be carried out prior to the final heat treatment or may be conducted as part of the final heat treatment e.g. during an initial, partial or pre-heating step.

The calcium-depleted MPC is preferably dried and then redissolved in the composition to be emulsified or in an aqueous component of it. Preferably, the MPC has at least 55% (on a moisture and fat-free basis), more preferably to at least 70% protein and most preferably to least 80% protein. The MPC preferably has 15-45%, more preferably 25-40%, most preferably 25-35% of the calcium replaced by monovalent cations, even more preferably 30-35% calcium replaced with monovalent cations, most preferably about 35%. This preference may vary depending on the formulation required by the end user. The preferred cations are sodium and potassium.

Where 15-45% of the calcium ions are removed, that is the percentage removed from the MPC relative to that from an MPC that has not been subjected to a calcium removal step. A standard MPC that is prepared at approximately neutral pH by ultrafiltration and diafiltration in this context has not been calcium depleted.

Liquid calcium-depleted MPCs (without drying) may also be used with the same protein and calcium concentration characteristics as defined for the dried ingredient.

The heat-treated calcium-depleted MPC, or the liquid nutritional composition, may be treated with an enzyme to further reduce the lactose concentration e.g. by a beta-galactosidase-treatment.

Preferably the calcium-depleted MPC is dried to a moisture content of less than 5%, or a water activity level that facilitates storage of the dry ingredient for several months without undue deterioration.

In some embodiments the calcium-depleted MPC of this invention may be blended with at least one other ingredient to produce a blend. Preferably the blend is a dry blend. Useful blends include blends of the calcium-depleted MPC with whey protein concentrates (WPCs).

Preferred MPCs for use in the invention have calcium that is manipulated by a cation exchange method. The manufacture and application of these calcium-depleted MPCs have been previously disclosed in US Patent 7,157,108, published PCT application WO2008/026940 and US published patent application 2010/0021595. These documents are fully incorporated herein by reference.

In those embodiments in which calcium manipulation is by acidification and subsequent dialysis and/or ultrafiltration and/or diafiltration, the pH is adjusted to be in the range 4.6-7.5, preferably 4.6-7.2, more preferably 4.6-6.7, most preferably 4.8-6.5. The membrane chosen generally has a nominal molecular weight cut off of 10,000 Daltons or less. A preferred ultrafiltration membrane is a Koch S4 HFK 131 type membrane with a nominal molecular weight cut off at 10,000 Daltons. The adjustment of the pH may be made with any acid suitable for adjusting the pH of a food or drink, e.g., dilute HCl, dilute H₂SO₄, dilute acetic acid, dilute lactic acid, preferably dilute citric acid. For this method it is preferred to neutralise the solution to obtain a pH of 6.4-7.0 after calcium manipulation and more preferably after ion exchange treatment. This neutralisation is preferably carried out before any drying step.

When the calcium manipulation is by way of addition of a chelating agent, preferred chelating agents for use include citric acid, EDTA, food phosphates/polyphosphates, food acidulants, tartaric acid, citrates and tartrates. The preferred chelating agents are food acidulating agents. The chelating agents may be used before, during or following ultrafiltration or diafiltration stages or independently of an ultrafiltration or diafiltration.

Preferably heat-treated calcium-depleted MPC comprises at least 51% by weight of the protein of the composition, preferably at least 70%, preferably at least 90%, most preferably 100%. Use of 100% is particularly advantageous as it gives the advantage of only a single easy to handle protein source being required.

The calcium-depleted MPC ingredient may be prepared from a mixture of MPCs, some with high levels of calcium depletion (e.g., 45-100%) and some with low levels or no depletion (e.g., 0-15%) as taught in US 7,157,108.

Other protein that may be included in amounts up to 49% include whey proteins, preferably provided from a whey protein concentrate comprising at least 50%, preferably at least 80%, of total solids as protein. Preferably the whey protein is prepared to minimise its tendency to gel (e.g., by methods as described in co-pending applications PCT/NZ2010/000072 and US 61/169,437).

The fat used may be vegetable fat or animal fat, including dairy fat and fish oils. Vegetable oils are often preferred because of their ease of formulation and lower saturated fatty acid content.

Preferred vegetable oils include canola (rapeseed) oil, corn oil, sunflower oil, olive or soybean oil.

The liquid nutritional composition may also include emulsifiers such as soya lecithin or phospholipids and the like in addition to the calcium-depleted MPC, which acts as an emulsifier as described in WO 2008/026940.

The carbohydrate used typically comprises digestible carbohydrate as 75-100% of the carbohydrate. The carbohydrate may comprise monosaccharides, disaccharides, oligosaccharides and polysaccharides and mixtures thereof. Oligosaccharides of glucose are typically used. A number of these are commercially available as maltodextrin (3-20 DE) or corn syrup for the longer chain carbohydrates (>20 DE). Non-digestible carbohydrates may also be included, for example, fructooligosaccharides, inulin, and galactooligosaccharides. These are typically present in amounts of 0.2-5% of the composition.

In preferred embodiments the liquid nutritional composition is a nutritionally complete composition or a high energy liquid or powder for breakfast or other times of the day.

In preferred embodiments the liquid nutritional composition contains nutrients that include vitamins and minerals. The recommended daily requirements of vitamins and minerals can be specified for various population subgroups. See for instance, Dietary Reference Intakes: RDA and AI for vitamins and elements, United States National Academy of Sciences, Institute of Medicine, Food and Nutrition Board (2010) tables recommended intakes for infants 0-6 , 6-12 months, children 1-3, and 4-8 years, adults males (6 age classes), females (6 age classes), pregnant (3 age classes) and lactating (3 age classes). Concentrations of essential nutrients in the liquid nutritional composition can be tailored in the preferred serve size for a particular subgroup or medical condition or application so that the nutrition and ease of delivery requirements can be met simultaneously.

The viscosity of the liquid enteral nutritional composition is preferably less than 150cP, more preferably less than 130cP, even more preferably less than 120cP, most preferably less than 90cP.

The pH of heat-treated calcium-depleted MPC is preferably 6.0-7.0, preferably 6.4-7.0, most preferably 6.8-7.0. The pH of the liquid nutritional composition is preferably 6.4-7.1, preferably 6.6-7.0, more preferably 6.8-7.0.

A particularly preferred liquid nutritional composition of the invention comprises 4-15% by weight heat-treated calcium-depleted MPC, 10-35% carbohydrate and 5-15% fat, wherein the nutritional composition has a viscosity of less that 200cP, an energy density of at least 0.5 kcal/ml, and wherein protein provides 10-40% of the total energy content of the composition, wherein the calcium-depleted MPC has undergone a heat treatment to at least 80°C and has 25-35% of the calcium replaced by potassium or sodium.

Also preferred is a powdered composition, dispersible in water to form a liquid nutritional composition comprising 4-15% by weight heat-treated calcium-depleted MPC, 10-35% carbohydrate and 5-15% by weight fat, wherein the nutritional composition has a viscosity of less that 200cP, an energy density of at least 0.5 kcal/ml, and wherein protein provides 10-40% of the total energy content of the composition, wherein the calcium-depleted MPC has undergone a heat treatment to at least 80°C and has 25-35% of the calcium replaced by potassium or sodium.

Also preferred is a liquid composition comprising 4-15% by weight heat-treated calcium-depleted MPC, 10-35% carbohydrate and 5-15% by weight fat, wherein the nutritional composition has a viscosity of less that 200cP, an energy density of at least 1.5 kcal/ml, and wherein protein provides 10-30% of the total energy content of the composition, wherein the calcium-depleted MPC has undergone a heat treatment to at least 80°C and has 25-35% of the calcium replaced by potassium or sodium.

Also preferred is a powdered composition, dispersible in water to form a liquid nutritional composition comprising 4-15% by weight heat-treated calcium-depleted MPC, 10-35% carbohydrate and 5-15% by weight fat, wherein the nutritional composition has a viscosity of less that 200cP, an energy density of at least 1.5 kcal/ml, and wherein protein provides 10-30% of the total energy content of the composition, wherein the calcium-depleted MPC has undergone a heat treatment to at least 80°C and has 25-35% of the calcium replaced by potassium or sodium.

The dry powder of the invention may be prepared by dry blending of the ingredients of the liquid nutritional composition. Alternatively a liquid nutritional composition may be dried, preferably by spray drying.

### Brief Description of the Drawing

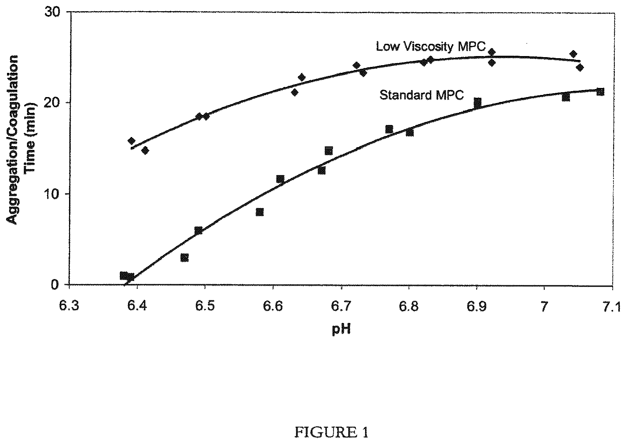

Figure 1 shows the heat stability (as determined by aggregation/coagulation time in minutes) of standard MPC (■) and Low Viscosity MPC (◆)(at 140°C) at pH values in the range 6.3-7.1.

### Examples

The following examples further illustrate practice of the invention.

Materials used in the following experiments are coded according to the detail below.
Standard MPC (MPC 4850, Fonterra Co-operative Group Ltd., Auckland, New Zealand)
MPC from this invention (Low Viscosity MPC, Fonterra Co-operative Group Ltd., Palmerston North, New Zealand). The compositions of the MPC ingredients are summarised in Table 1.
Corn Oil -supplied by NZ Bakels Ltd., Auckland, New Zealand
Lecithin - supplied as Topcithin NGM Liquid soy lecithin - by Cargill, Incorporated Minneapolis, MN, United States
Maltodextrin - supplied as MALTRIN M180 (Dextrose Equivalent 18.5) - Grain Processing Corporation, Iowa, USA.
Sucrose - supplied as Chelsea Extra Fine Sugar - Manufacturer: NZ Sugar Co Ltd., Auckland, New Zealand.

**Table 1 Summary of Composition of MPCs used**

| Material | Standard MPC | Low Viscosity MPC |
|---|---|---|
| Protein (%) | 83 | 83 |
| Calcium (%) | 2.2 | 1.5 |
| Sodium (%) | 0.08 | 1.0 |

### Example 1 Heat Stability of Low Viscosity MPC in Aqueous Solution

Three batches of 35%-calcium depleted MPC retentate were prepared as described in US7157108 and designated Low-viscosity MPC85. In each case, the calcium depleted MPC retentate was heat treated at a temperature/time combination of 90°C/4 s and then evaporated and dried to produce Low-viscosity MPC85 ingredient which was used in all the examples.

A 5% protein solution of either standard or the Low-viscosity MPC85 powder was stirred at 60°C and left to hydrate for 30 minutes. The solution was sub-sampled in 30mL batches and pH adjusted in the range 6.3 -7.1. For heat stability testing, 1mL aliquots of the pH adjusted solutions were transferred to glass tubes. The glass tubes were placed in an oil bath at 140°C and visually observed for aggregation and/or coagulation.

### Material/Apparatus

Water bath controlled at 60±1°C
Stainless steel beakers (500 mL)
Mechanical stirrers and blades
Weighing boats or small beakers
A timer
Transfer pipettes
Analytical balance weighing to 4 decimal places
1-200 µL, 1mL, and 10 mL pipettes
Magnetic stirrers
pH meter
50mL sample jars
Reagents 1M HCl & 1M NaOH
Oil bath at 140°C for heat stability testing
8 mL heat resistant glass sample tubes
Heatproof gloves or tongs for handling

### Procedure

1. A water bath was preheated to 60°C.
2. The weight of a stainless steel beaker was weighed and recorded.
3. Weigh in the required amount of demineralised water for a 5% protein solution totalling 400 g.
4. A beaker was placed into water bath under an overhead stirrer and the contents allowed to warm to 60°C.
5. 24 g of protein powder was weighed (taking into account total % protein in powder) for a 5% protein solution totalling 400 g.
6. The water was stirred to a deep vortex and the powder slowly added.
7. Once all the powder had been added, the mixing speed was slowed down and the mixture allowed to hydrate for 30 mins.
8. The stirrer was stopped and the beaker and contents were reweighed after the 30 min hydration. The solution was topped up to 400g using RO water and mixed thoroughly. 30 mL aliquots of the solution were sub-sampled into sample containers.
9. Each sample was pH adjusted in the range 6.3 - 7.1 using 1M HCl or NaOH with constant stirring. The oil bath was preheated to 140°C.
10. 1mL of each sample was transferred into a clear glass tube for heat stability testing. Care was taken to ensure that the samples did not touch the walls of the sample jar and were placed directly on the bottom.
11. The samples in the oil bath were held at 140°C using the shaker. As soon as the samples were placed in the oil bath the timer and the shaker were started.
12. The samples were observed visually and the time for aggregation/coagulation of the sample recorded.

The results are shown in Figure 1 and show that the Low Viscosity MPC had greater stability to high temperatures at the pHs tested than the non inventive control.

### Example 2: Performance of Low Viscosity MPC in a Model Formulation - UHT Process

Three batches of the model nutritional formulation were prepared according to the method detailed below, using the three samples of the Low-viscosity MPC85 powder described in Example 1 and these were designated Trials 1, 2 & 3.
1. 60°C demineralised water (36 kg) was weighed into the jacketed mixing vessel
2. Protein powder (7.1 kg Low-viscosity MPC85) was added into continuously stirred water and hydrated for 60 minutes whilst stirring continuously.
3. Carbohydrate blend (18.2 kg) comprising maltodextrin (5.7 kg) and sucrose (12.5 kg) was added and mixed.
4. The mineral blend including potassium chloride (56.6 g), potassium citrate (271.9 g), magnesium chloride (126.8 g) and calcium phosphate (4.6 g) was pre-dissolved in a small amount of water. The mineral solution was then added to the ingredients in the jacketed mixing vessel and mixed.
5. The vegetable oil (5.5 kg) was heated in a separate container to 60°C. The soy lecithin (91 g) was warmed and then added to the oil and this mixture was transferred to the other ingredients already in the jacketed mixing vessel followed by mixing for 5 min.
6. The prepared mixture in the jacketed mixing vessel was passed through a two-stage homogeniser (200/50 Bar).
7. The homogenised mixture was cooled to 25°C and the pH adjusted to target pH 6.8 with KOH.
8. The mixture was UHT processed at about 145°C for 4 or 5 sec at a product flow rate of 120 1/hr and then packed aseptically. The preheating temperature was between 83-85°C and achieved using a plate heat exchanger. The product was held for 30 seconds at this temperature. The final heat treatment temperature was then raised to 144-145°C using direct steam injection. After the holding tube, the first stage of cooling was to 86-87°C using a flash vessel and the final cooling was to about 24-25°C using a plate heat exchanger. The product was immediately packed at about 24-25°C into 250 mL glass bottles and capped.
9. The viscosity, pH and particle size of the final product were measured within 7 days of the UHT heat treatment. The remaining samples were placed into storage at 30°C for several months and evaluated at 1, 3, 6 & 9 months. Viscosity was measured at 20°C at 100 s⁻¹ using Anton Paar Physica MCR301 rheometer fitted with cup and bob. The results of the tests are summarised in Table 2. The pH was measured using standard methods at 20°C and the mean particle size (characterised by [D4,3]) was determined by laser diffraction using a Malvern particle size analyser (Mastersizer 2000, Malvern Instruments Ltd, Malvern, United Kingdom).

The viscosity results are compared in Table 2. The results show that after heat treatment the viscosities were less than 75 cP at 20°C for the 3 repeated trials.

**Table 2 Results of storage of UHT treated samples held at 30°C**

| | Initial | 1 Month | 3 Months | 6 Months | 9 Months |
|---|---|---|---|---|---|
| | pH | | | | |
| Trial 1 | 6.81 | 6.72 | 6.71 | 6.59 | 6.53 |
| Trial 2 | 6.82 | 6.71 | 6.70 | 6.59 | 6.53 |
| Trial 3 | 6.83 | 6.72 | 6.71 | 6.60 | 6.54 |
| | Viscosity (cP) | | | | |
| Trial 1 | 55 | 55 58 | | 61 | 62 |
| Trial 2 | 56 | 57 | 58 | 63 | 65 |
| Trial 3 | 62 | 63 | 63 | 69 | 73 |
| | **Mean particle size [D4,3] (µm)** | | | | |
| Trial 1 | 1.5 | 1.0 | 0.6 | 0.77 | 0.99 |
| Trial 2 | 0.55 | 0.49 | 0.50 | 0.56 | 0.51 |
| Trial 3 | 0.58 | 0.64 | 0.60 | 0.58 | 0.60 |

After 9 months storage at 30°C the cream and sedimentation layers were assessed visually as being slight. All samples were commercially acceptable.

### Example 3 Performance of Low Viscosity MPC in a Model Formulation - Retort Process

Three samples of the model nutritional formulation were agam prepared from the batches of Low viscosity MPC85 powder described above. In this example, steps 1-7 and 9 were as in Example 2 except that after homogenisation at step 7, the samples were packed into 210 mL cans were filled and sealed. Step 8 used retorting instead of UHT as the heat treatment. In the retort the sterilisation treatment of the filled cans was 121°C for 10 minutes. The retort used a pressurised steam heat treatment followed by full immersion water cooling to 50°C, then cooling was completed by ambient storage.

The results of the tests are summarised in Table 3. They show that after heat treatment the viscosities were less than 120 cP at 20°C for the 3 repeated trials.

**Table 3 Results of storage of retorted samples held at 30°C**

| | Initial | 1 Month | 3 Months | 6 Months | 9 Months |
|---|---|---|---|---|---|
| | pH | | | | |
| Trial 1 | 6.69 | | 6.55 | 6.40 | 6.41 |
| Trial 2 | 6.66 | | 6.51 | 6.40 | 6.39 |
| Trial 3 | 6.64 | | 6.55 | 6.41 | 6.39 |
| | Viscosity (cP) | | | | |
| Trial 1 | 98 | | 115 | | 126 |
| Trial 2 | 103 | | 115 | | 132 |
| Trial 3 | 114 | | 114 | | 141 |
| | **Mean particle size [D4,3] (µm)** | | | | |
| Trial 1 | | | 0.87 | | 0.90 |
| Trial 2 | | | 0.82 | | 0.81 |
| Trial 3 | | | 0.77 | | 0.88 |

After 9 months storage at 30°C the cream and sedimentation layers were assessed visually as being slight. All samples were commercially acceptable.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

In this specification, percentages are on a by weight basis, unless the context indicates otherwise.

It is not the intention to limit the scope of the invention to the abovementioned examples only. As would be appreciated by a skilled person in the art, many variations are possible without departing from the scope of the invention. For example, the percentage protein and the calcium-depletion of the MPC can be varied, as can the nature and proportions of the other components of the nutritional composition.

### REPRESENTATIVE ASPECTS

1. A liquid nutritional composition comprising (a) 2-25% by weight of a calcium-depleted milk protein concentrate (MPC) that has undergone a heat treatment to at least 80°C and has between 15-45% by weight of the calcium removed; (b) 0-30% by weight fat; (c) 5-45% by weight carbohydrate; wherein the nutritional composition has a viscosity of less than 200cP at a temperature of 20°C and shear rate of 100s⁻¹, and has an energy density of at least 0.5 kcal/ml, and wherein protein provides 10-40% of the total energy content of the composition.
2. A powdered nutritional composition dispersible in water to form a liquid nutritional composition comprising (a) 2-25% by weight of a calcium-depleted milk protein concentrate (MPC) that has undergone a heat treatment to at least 80°C and has between 15-45% by weight of the calcium removed; (b) 0-30% by weight fat; (c) 5-45% by weight carbohydrate; wherein the nutritional composition has a viscosity of less than 200cP at a temperature of 20°C and shear rate of 100s⁻¹, and has an energy density of at least 0.5 kcal/ml, and wherein protein provides 10-40% of the total energy content of the composition.
3. A composition as claimed in aspect 1 or aspect 2 where the calcium depleted MPC has undergone a heat treatment comprising heating to 80-140°C.
4. A composition as claimed in aspect 1 wherein the liquid nutritional composition is heated to a temperature above 100°C.
5. A composition as claimed in aspect 1 or aspect 4 wherein the composition comprises 4-25% by weight of heat-treated calcium depleted MPC.
6. A composition as claimed in any one of aspects 1, 4 and 5 wherein the composition comprises 5-25% by weight of fat.
7. A composition as claimed in any one of aspects 1 and 4-6 wherein the carbohydrate content is 5-40% by weight.
8. A composition as claimed in any one of aspects 1-7 wherein the calcium depleted MPC has 25-40% of the calcium replaced with potassium or sodium.
9. A composition as claimed in any one of aspects 1-8 wherein the MPC (on a moisture and fat-free basis) comprises at least 70% protein.
10. A composition as claimed in any one of aspects 1-9 wherein the calcium depletion of the MPC has been by cation exchange with the potassium or sodium.
11. A liquid nutritional composition as claimed in aspect 1 comprising 4-15% by weight heat-treated calcium-depleted MPC, 10-35% carbohydrate and 5-15% fat, wherein the nutritional composition has a viscosity of less that 200cP, an energy density of at least 0.5 kcal/ml, and wherein protein provides 10-40% of the total energy content of the composition, wherein the calcium-depleted MPC has undergone a heat treatment to at least 80°C and has 25-35% of the calcium replaced by potassium or sodium.
12. A powdered nutritional composition as claimed in aspect 2, dispersible in water to form a liquid nutritional composition comprising 4-15% by weight heat-treated calcium-depleted MPC, 10-35% carbohydrate and 5-15% fat, wherein the nutritional composition has a viscosity of less that 200cP, an energy density of at least 0.5 kcal/ml, and wherein protein provides 10-40% of the total energy content of the composition, wherein the calcium-depleted MPC has undergone a heat treatment to at least 80°C and has 25-35% of the calcium replaced by potassium or sodium.
13. A composition as claimed in aspect 2 wherein the ingredients of the liquid nutritional composition are dry blended.
14. A spray dried composition formed by spray drying a composition as claimed in aspect 1.
15. A liquid nutritional composition as claimed in aspect 1 wherein the calcium removed from milk protein concentrate (MPC) has been replaced by sodium or potassium; and the energy density is at least 1.5 kcal/ml, and protein provides 10-30% of the total energy content of the composition.
16. A powdered nutritional composition as claimed in aspect 2 that is dispersible in water to form a liquid nutritional composition comprising (a) 2-25% by weight of a calcium-depleted milk protein concentrate (MPC) that has undergone a heat treatment to at least 80°C and has between 15-45% by weight of the calcium replaced by sodium or potassium; (b) 0-25% by weight fat; (c) 5-45% by weight carbohydrate; wherein the nutritional composition has a viscosity of less than 200cP, and has an energy density of at least 1.5 kcal/ml, and protein provides 10-30% of the total energy content of the composition.
17. A composition as claimed in aspect 15 or aspect 16 where the calcium depleted MPC has undergone a heat treatment comprising heating to 80-140°C.
18. A composition as claimed in aspect 15 wherein the liquid nutritional composition is heated to a temperature above 100°C.
19. A composition as claimed in aspect 15 or aspect 18 wherein the composition comprises 4-20% by weight of heat-treated calcium depleted MPC.
20. A composition as claimed in any one of aspects 15, 18 and 19 wherein the composition comprises 5-25% by weight of fat.
21. A composition as claimed in any one of aspects 15 and 18-20 wherein the carbohydrate content is 5-40% by weight.
22. A composition as claimed in any one of aspects 15-21 wherein the calcium depleted MPC has 25-40% of the calcium replaced with potassium or sodium.
23. A composition as claimed in any one of aspects 15-22 wherein the MPC (on a moisture and fat-free basis) comprises at least 70% protein.
24. A composition as claimed in any one of aspects 15-23 wherein the calcium depletion of the MPC has been by cation exchange with the potassium or sodium.
25. A composition as claimed in aspect 1 comprising 4-15% by weight heat-treated calcium-depleted MPC, 10-35% carbohydrate and 5-15% by weight fat, wherein the nutritional composition has a viscosity of less that 200cP, an energy density of at least 1.5 kcal/ml, and wherein protein provides 10-30% of the total energy content of the composition, wherein the calcium-depleted MPC has undergone a heat treatment to at least 80°C and has 25-35% of the calcium replaced by potassium or sodium.
26. A powdered nutritional composition as claimed in aspect 16, dispersible in water to form a liquid nutritional composition comprising 4-15% by weight heat-treated calcium-depleted MPC, 10-35% carbohydrate and 5-15% by weight fat, wherein the nutritional composition has a viscosity of less that 200cP, an energy density of at least 1.5 kcal/ml, and wherein protein provides 10-30% of the total energy content of the composition, wherein the calcium-depleted MPC has undergone a heat treatment to at least 80°C and has 25-35% of the calcium replaced by potassium or sodium.
27. A composition as claimed in aspect 26 wherein the ingredients of the liquid nutritional composition are dry blended.
28. A spray dried composition formed by spray drying a composition as claimed in aspect 25.
29. A composition as claimed in any one of aspects 1-28 wherein the liquid nutritional compositions is heated at 140°C-150°C for 2-5 seconds or at 120°C-130°C for 10-20 minutes.

This divisional application is divided from EP11807119.0, the European regional phase of PCT/NZ2011/000134 filed on 15.07.2011 at the NZ patent office (the 'parent application') and the divisional specification as filed comprises the content of the parent application, including, but not limited to, the description, any drawings, any sequence listing(s) and the original claims recited as 'representative aspects'. The scope of this disclosure therefore includes the full content of the parent application. In any case, protection may be sought for any features disclosed in the parent application as filed.

## Claims

1. A liquid nutritional composition comprising
(a) 2-25% by weight of a calcium-depleted milk protein concentrate (MPC) that has undergone a heat treatment to at least 80°C and has between 15-45% by weight of the calcium removed;
(b) 0-30% by weight fat;
(c) 5-45% by weight carbohydrate;
wherein the liquid nutritional composition has a viscosity of less than 0.2 Pa.s (200cP) at a temperature of 20°C and shear rate of 100s⁻¹, and has an energy density of at least 0.5 kcal/ml, and wherein protein provides 10-40% of the total energy content of the composition.

2. A composition as claimed in claim 1 wherein the calcium depleted MPC has undergone a heat treatment to at least 80°C for 1 second to 20 minutes, or undergone a heat treatment to 80-140°C for 1 second to 20 minutes, or undergone a heat treatment to 80-120°C for about 1 second to 20 minutes, during preparation of the calcium-depleted MPC.

3. A composition as claimed in claims 1 or 2 wherein the liquid nutritional composition has undergone a heat treatment to increase storage time of the product.

4. A composition as claimed in any one of claims 1 to 3 wherein the liquid nutritional composition is heated to a temperature above 100°C, or
the liquid nutritional composition is heated at 140°C-150°C for 2-5 seconds or at 120°C-130°C for 10-20 minutes. or
the liquid nutritional composition has undergone ultra-high heat treatment (UHT), or
the liquid nutritional composition has undergone retort heat treatment,

5. A composition as claimed in any one of claims 1 to 4 wherein the composition comprises 4-25% by weight of heat-treated calcium depleted MPC, or 4-20% by weight, of a calcium-depleted milk protein concentrate (MPC).

6. A composition as claimed in any one of claims 1 to 5 wherein the composition comprises 5-25% by weight of fat.

7. A composition as claimed in any one of claims 1 to 6 wherein the carbohydrate content is 5-40% by weight.

8. A composition as claimed in any one of claims 1 to 7 wherein the calcium depleted MPC has 25-40% of the calcium replaced with potassium or sodium, and/or wherein the calcium depletion of the MPC has been by cation exchange with the potassium or sodium.

9. A composition as claimed in any one of claims 1 to 8 wherein the MPC (on a moisture and fat-free basis) comprises at least 70% protein.

10. A composition as claimed in any one of claims 1 to 9 comprising 4-15% by weight heat-treated calcium-depleted MPC, 10-35% carbohydrate and 5-15% fat, wherein the nutritional composition has a viscosity of less than 0.2 Pa.s (200cP), an energy density of at least 0.5 kcal/ml, and wherein protein provides 10-40% of the total energy content of the composition, wherein the calcium-depleted MPC has undergone a heat treatment to at least 80°C and has 25-35% of the calcium replaced by potassium or sodium.

11. A composition as claimed in any one of claims 1 to 10 wherein the calcium removed from milk protein concentrate (MPC) has been replaced by sodium or potassium; and the energy density is at least 1.5 kcal/ml, and protein provides 10-30% of the total energy content of the composition.

12. A composition as claimed in any one of claims 1 to 11 comprising 4-15% by weight heat-treated calcium-depleted MPC, 10-35% carbohydrate and 5-15% by weight fat, wherein the nutritional composition has a viscosity of less than 0.2Pa.s (200cP), an energy density of at least 1.5 kcal/ml, and wherein protein provides 10-30% of the total energy content of the composition, wherein the calcium-depleted MPC has undergone a heat treatment to at least 80°C and has 25-35% of the calcium replaced by potassium or sodium.

13. A powder composition formed by drying a composition as claimed in any one of claims 1 to 12, optionally wherein the drying is spray drying.

14. A method for preparing a liquid nutritional composition comprising
(a) preparing a heat-treated calcium-depleted milk protein concentrate (MPC), the preparation comprising calcium depleting an MPC by 15-45% by weight of the calcium and heating the calcium-depleted MPC to at least 80°C;
(b) dispersing 2-25% by weight of the heat-treated calcium-depleted MPC in water with 0-30% by weight fat and 5-45% by weight carbohydrate;
(c) heating the liquid nutritional composition;
wherein the liquid nutritional composition has a viscosity of less than 0.2 Pa.s (200cP) at a temperature of 20°C and shear rate of 100s⁻¹; and
wherein the liquid nutritional composition has an energy density of at least 0.5 kcal/ml, and wherein protein provides 10-40% of the total energy content of the liquid nutritional composition.

15. A powdered nutritional composition dispersible in water to form a liquid nutritional composition comprising (a) 2-25% by weight of a calcium-depleted milk protein concentrate (MPC) that has undergone a heat treatment to at least 80°C and has between 15-45% by weight of the calcium removed; (b) 0-30% by weight fat; (c) 5-45% by weight carbohydrate; wherein the nutritional composition has a viscosity of less than 0.2 Pa.s (200cP) at a temperature of 20°C and shear rate of 100s⁻¹, and has an energy density of at least 0.5 kcal/ml, and wherein protein provides 10-40% of the total energy content of the composition.
